# EUROPÄISCHE PATENTANMELDUNG

(11) **EP 0 791 327 A2**
(43) Veröffentlichungstag der Anmeldung: **27.08.1997**
(21) Anmeldenummer: 97102580.4
(22) Anmeldetag: 18.02.1997
(51) Int. Cl.: A61B 5/091

(54) **Verfahren und Vorrichtung zur Bestimmung der funktionellen Residualkapazität (FRC)**

(30) Priorität: 21.02.1996 DE 19606470
(71) Anmelder: MPO Gesellschaft für Medizintechnische Produkt Organisation mbH, 83229 Aschau (DE)
(72) Erfinder: Hecker, Karl-Heinz, 83229 Aschau (DE); Schinagl, Rudolf, Dipl.-Ing., 82008 Unterhaching (DE); Wagner, Thomas O.F., Prof. Dr., 30175 Hannover (DE)
(74) Vertreter: Rost, Jürgen, Dipl.-Ing.

(57) **Zusammenfassung**

Die Erfindung betrifft ein Verfahren und eine Vorrichtung zur Bestimmung der funktionellen Residualkapazität (FRC) durch Einspülung von Helium oder eines anderen inerten Gasgemischs. Erfindungsgemäß wird an das Mundstück eines Tubus oder an eine Maske bei der Zwangsbeatmung eines Patienten über mehrere Atemzyklen durch eine Meßapparatur die Dichte des Gasgemischs bei der Inspiration und bei der Expiration gemessen. Aus der Differenz der Gaskonzentrationen wird die FRC bestimmt.

## Beschreibung

Die Erfindung betrifft ein Verfahren zur Bestimmung der funktionellen Residualkapazität (FRC) mit den im Oberbegriff des Anspruchs 1 angegebenen Merkmalen.

Um eine den Patienten schonende Beatmung durchführen zu können, ist es notwendig, die FRC des Patienten zu kennen. Dadurch können die Beatmungsparameter auf den Patienten abgestimmt werden, was zur Folge hat, daß der Patient während der Beatmung weniger Schaden erleidet, als wenn mit einer zu kleinen oder zu großen FRC gearbeitet wird. Die physiologisch abnormale Beatmung wird so entschärft, und es wird auch die Entwöhnung von der Beatmung wesentlich verkürzt.

Es ist bereits bekannt, die FRC-Bestimmung durch oszillatorische Dichtemessung vorzunehmen, was aber bei den bisher bekannten Verfahren nur in einem geschlossenen System und damit nicht während der maschinellen Beatmung durchgeführt werden kann.

Aus der DE 29 12 391 B2 ist bekannt, daß zur Lungenfunktionsanalyse ein Gerät mit einem Atembeutel verwendet wird, wobei der Proband an den Atembeutel angeschlossen wird und bei Hin- und Rückatmung eines Helium enthaltenden Testgasgemisches die Dichteänderung des Gasgemisches im Atembeutel gemessen und daraus auf die FRC geschlossen wird. Es handelt sich hier um ein geschlossenes System, das dadurch gekennzeichnet ist, daß der Proband in einen nach außen geschlossenen Beutel atmet und aus diesem Beutel auch wieder einatmet. Für die FRC-Bestimmung wird der Proband an den Atembeutel angeschlossen und nach relativ kurzer Rückatmungszeit von diesem System wieder getrennt. Mit dem bekannten System ist eine FRC-Bestimmung während der maschinellen Beatmung nicht möglich.

In der US 4,221,224 ist beschrieben, daß bei einem Verfahren zur Bestimmung der Alveolar-Ventilation der inspiratorische Teil und der exspiratorische Teil des Systems voneinander getrennt sind. Mit der bekannten Methode wird nicht die FRC, sondern es werden Gaswerte im Blut bestimmt.

Der Erfindung liegt die Aufgabe zugrunde, ein Verfahren zur Bestimmung der FRC anzugeben, das auch während der maschinellen Beatmung durchgeführt werden kann.

Die Lösung dieser Aufgabe wird verfahrensmäßig mit den im Anspruch 1 angegebenen Merkmalen, vorrichtungsmäßig mit den im Anspruch 6 angegebenen Merkmalen erreicht.

Dadurch, daß die Bestimmung der FRC in einem offenen System erreicht wird, kann das Verfahren während der maschinellen Beatmung durchgeführt werden, und zwar ohne diese zu beeinflussen und die vorgewählten Parameter am Respirator ändern zu müssen. Unter einem offenen System ist dabei zu verstehen, daß die Ausatemluft ins Freie gelangt und dem Probanden bei jedem Atemzug ein neues Gasgemisch zugeführt wird. Zur FRC-Bestimmung wird am Ende eines Atemzyklus die definierte Zugabe von Helium gestartet. Die Helium-Konzentration wird bei jedem Atemzyklus inspiratorisch und exspiratorisch ermittelt. Die Differenz an Helium wird registriert und über so viele Atemzyklen integriert, bis die Differenz einen vorgewählten Grenzwert unterschreitet. Aus der Summe der in der Lunge des Patienten verbleibenden Heliummengen wird die Größe der FRC bestimmt. Statt Helium kann auch ein anderes inertes Gas verwendet werden. Die Restgasmenge enthält mindestens 21 % Sauerstoff.

Vorteilhafte weitere Ausgestaltungen des Verfahrens nach Anspruch 1 sind Gegenstand der Unteransprüche 2 bis 5, weitere vorteilhafte Ausgestaltungen der Vorrichtung nach Anspruch 6 sind Gegenstand der Unteransprüche 7 bis 9.

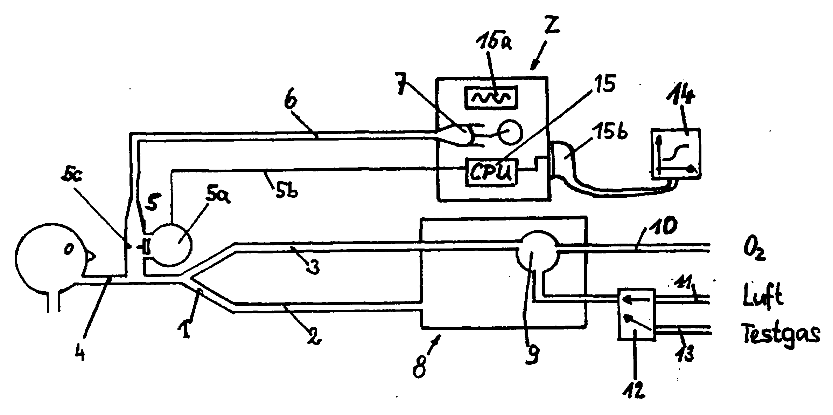

Im folgenden wird die Erfindung anhand eines in der Zeichnung dargestellten Ausführungsbeispiels näher erläutert. Die einzige Zeichnungsfigur zeigt schematisch eine Vorrichtung zur Messung der RFC bei einem beatmeten Patienten.

Mit Hilfe eines Respirators 8 wird ein Patient beatmet. Zur Beatmung wird an den Respirator 8 über den Anschluß 10 Sauerstoff und über den Anschluß 11 Luft eingespeist. Am Anschluß 11 kann mittels eines Ventils 12 von Luft auf Testgas umgeschaltet werden, welches über einen Anschluß 13 zugeführt wird.

Das im Respirator 8 mittels eines Mischventils 9 homogen gemischte Beatmungsgas wird über den Inspirationsschlauch 3 und das Y-Stück 1 dem Mundstück 4 des Tubus zugeführt. Bei der Exhalation fließt der Ausatemstrom über das Y-Stück 1 und den Exspirationsschlauch 2 in den Respirator 8 zurück.

In eine Meßapparatur 5 mit einer Stenose 5c und einem Drucksensor 5a wird mit einer oszillierend arbeitenden Pumpe 7 über eine Druckleitung 6 eine kleine Menge Luft gepumpt, und die oszillierenden Druckschwankungen werden dort gemessen. Der Meßwert wird über die Datenleitung 5c der CPU 15 zugeführt und dort ausgewertet. Auf einem Display 15a wird das Ergebnis angezeigt. Über entsprechende Schnittstellen 15b kann ein Schreiber 14 oder ein Zentralcomputer angeschlossen werden.

Helium hat abweichend von den in der Luft vorkommenden Gasen eine wesentliche geringere Dichte. Dieses bewirkt, daß bei einer Beimischung von Helium zur Luft die Dichte des Gasgemisches linear abhängig ist von der Heliumkonzentration. Gleichzeitig bewirkt die Dichteänderung eine höhere oder niedrigere Viskosität des Gasgemisches, je nach dem, wieviel Helium der Luft beigemischt wurde.

Wird das Gasgemisch mit der Pumpe 7 mit gleichförmigem Fluß durch die Stenose (Blende) 5c gepreßt, so stellt sich abhängig von der Viskosität des Gases eine Druckdifferenz über die Stenose 5c ein. Diese wird mit einem Drucksensor 5a gemessen und der CPU 15 über die Datenleitung 5b zugeführt. Die Zugabe von Helium wird endexspiratorisch begonnen. Mit dem Respirator 8 wird der Patient mit einem Beatmungsgas mit einer definierten Konzentration an Helium beatmet. Diese Heliumkonzentration wird gemessen. In der Lunge wird sich das Helium auch auf den nicht bei der Exspiration entleerten Teil der Lunge, die FRC, ausbreiten. Bei der Exhalation hat daher das Ausatemgas eine andere, geringere Konzentration an Helium. Diese wird wieder mit der Meßapparatur 5 gemessen, und das Ergebnis wird über die Datenleitung 5b der CPU 15 zugeführt.

Die Differenz der Helium-Konzentration wird in einem Speicher der CPU 15 abgelegt und addiert.

Nach etwa zehn Beatmungszyklen ist die Heliumkonzentration bei Inspiration und Exspiration ausgeglichen. Aus der Summe der Heliumkonzentrationsdifferenzen errechnet die CPU 15 die absolute Menge an Helium, die in den nicht entleerten Bereich der Lunge eingespült wurde. Aus dieser aboluten Menge Helium und aus der Heliumkonzentration des Beatmungsgases errechnet die CPU 15 die funktionelle Residualkapazität FRC.

## Patentansprüche

1. Verfahren zur Bestimmung der funktionellen Residualkapazität (FRC) und anderer Lungenvolumina durch Einspülung von Helium oder einem anderen inerten Gas unter Verwendung einer oszillatorischen oder anderen Bestimmung,
**dadurch gekenzeichnet**,
daß einem Patienten oder Probanden in einem offenen System über einen Respirator oder ein anderes, die Atmung unterstützendes Gerät ein Gasgemisch zugeführt wird, das Helium oder ein anderes inertes Gas enthält, und dabei durch eine am Tubus oder an der Maske angesetzte Meßapparatur die Konzentration und Menge dieses Gasgemisches und damit die Fremdgas- oder Heliumkonzentration gemessen wird,
daß bei dem exspiratorischen Gasgemisch ebenfalls die Konzentration oder Dichte des Gasgemischs gemessen und über den Vergleich der Meßwerte der Gasgemische beim Ein- bzw. Ausatmen das in der Lunge des Patienten zu messende Volumen bestimmt wird,
daß diese Schritte so lange wiederholt werden, bis bei einem Atemzyklus die Differenz zwischen inspiratorischer und exspiratorischer Gaskonzentration einen vorgewählten Grenzwert unterschreitet oder aus dem Verlauf der Gaskonzentrationen der weitere Verlauf der Gaskonzentrationsänderung vorhergesagt werden kann.

2. Verfahren nach Anspruch 1, dadurch gekennzeichnet, daß einem im Respirator angeordneten Mischventil über eine Zuführleitung 100 % Sauerstoff und über eine zweite Zuführleitung wahlweise entweder ein einen relativ hohen Helium-Anteil enthaltendes Testgas oder Atmosphärenluft zugeführt wird.

3. Verfahren nach Anspruch 1 oder 2, dadurch gekennzeichnet, daß als Meßapparatur ein Drucksensor eingesetzt wird und die von diesem gemessenen Werte gesammelt und in einem Algorithmus verarbeitet werden.

4. Verfahren nach Anspruch 3, dadurch gekennzeichnet, daß das Ergebnis der Messung der FRC sichtbar gemacht wird.

5. Verfahren nach einem der Ansprüche 1 bis 4, dadurch gekennzeichnet, daß an die Meßapparatur eine mit einer Frequenz von etwa 10 Hz oszillierend arbeitende Pumpe angeschlossen wird.

6. Vorrichtung zur Durchführung des Verfahrens nach einem der Ansprüche 1 bis 5, dadurch gekennzeichnet, daß am Mundstück (4) eines Tubus oder an einer Maske, über den bzw. über die der beatmete Patient oder Proband mit einem Respirator (8) verbunden ist, über eine Meßapparatur (5) ein Zusatzgerät (Z) anschließbar ist, in dem die von der Meßapparatur (5) ermittelten Dichten des Gasgemischs beim Ein- bzw. Ausatmen in einem CPU (15) ausgewertet werden.

7. Vorrichtung nach Anspruch 6, dadurch gekennzeichnet, daß das Zusatzgerät (Z) eine oszillierend arbeitende Pumpe (7) aufweist, die über eine Druckleitung (6) mit der Meßapparatur (5) verbunden ist.

8. Vorrichtung nach Anspruch 6 oder 7, dadurch gekennzeichnet, daß die Meßapparatur (5) einen Drucksensor (5a) aufweist, der mit einer Stenose (5c) ausgestattet ist und von dem die Meßwerte über eine Datenleitung (5b) dem Zusatzgerät (Z) zugeführt werden.

9. Vorrichtung nach einem der Ansprüche 6 bis 8, dadurch gekennzeichnet, daß an das Zusatzgerät (Z) über Schnittstellen (15b) ein Schreiber (14) oder ein Zentralcomputer angeschlossen ist.
